# EUROPEAN PATENT APPLICATION

(11) **EP 2 826 814 A1**
(43) Date of publication of application: **21.01.2015**
(21) Application number: 13177153.7
(22) Date of filing: 19.07.2013
(51) Int. Cl.: C08J 9/26, B29C 67/00, B29C 44/12, B29C 67/20, A61L 27/56

(54) **Method of Manufacturing A Porous Polymer Component Involving Use of A Dissolvable, Sacrificial Material**

(71) Applicant: Danmarks Tekniske Universitet, 2800 Lyngby (DK)
(72) Inventor: Mohanty, Soumyaranjan, 2850 Nærum (DK); Emnéus, Jenny, 211 55 Malmø (SE); Wolff, Anders, 2100 Copenhagen Ø (DK); Dufva, Martin, 3100 Hornbæk (DK); Muhammad, Haseena Bashir, 2100 Copenhagen Ø (DK); Skolimowski, Maciej, 2800 Kgs. Lyngby (DK); Amato, Letizia, 2200 Copenhagen N (DK)
(74) Representative: Plougmann & Vingtoft A/S

(57) **Abstract**

The present invention relates to a method of manufacturing a porous polymer component 1 with structured and/or random pores 4 and/or channels 5. The method comprises arranging a dissolvable, sacrificial material 2 in a geometrical arrangement corresponding to an inner structure to be obtained in the polymer component 1. A component material 3, which is to form the final component 1, is arranged so that it surrounds at least a majority of the sacrificial material 2, and subsequently the sacrificial material 2 is dissolved and removed from the component material 3. The sacrificial material 2 and thereby the resulting inner structure of the component 1 is arranged in a controlled and reproducible manner. The sacrificial material 2 and possibly also the component material 3 may e.g. be arranged by use of a 3D-printer or manually. The method may e.g. be used to manufacture a three-dimensional scaffold for tissue engineering.

## Description

### FIELD OF THE INVENTION

The present invention relates to the manufacturing of a porous polymer component, and in particular to methods involving arrangement of a sacrificial material which is subsequently dissolved and removed from a component material in order to form the cavities in the inner structure of the component.

### BACKGROUND OF THE INVENTION

Porous polymers are used for a very large number of applications for years including as different ones as: core materials in lightweight engineering structures, thermal insulation, packaging and various products for personal care. The microstructure of the porous polymers is typically random, and the main purpose of the material typically relates to the "build-in" air bubbles being formed as part of the manufacturing process. However, for some applications it would be desirable to be able to control the microstructure of the polymer, e.g. to ensure that a fluid can pass through the material in a controllable manner.

In the field of tissue engineering, there is a requirement for porous material scaffolds that can be used to integrate biological cells or molecules to regenerate tissues. Such scaffolds support the spatial distribution of cells in a three dimensional structure, provide mechanical stability to the cells and should enable optimum nutrient transport. The desired scaffold would be highly porous and have interconnected pores. Thus, there is a need for reproducible and fabricatable scaffolds with controlled porosity.

Attempts have been made to make a porous polymer material by use of a sacrificial material constituting what are to become cavities in the final product when the sacrificial material is removed after moulding a polymer around it. US 6,900,055 discloses a method of manufacturing a porous silicone rubber for growing cells or living tissue. A silicone rubber precursor is contacted with biologically acceptable sacrificial filler; this filler is described as a ground inorganic salt. The inner structure of the porous polymer thus reproduces the structure of the filler and can only be selected by choosing the best available sacrificial filler material for a given application.

Hence, an improved method of manufacturing a porous polymer component would be advantageous.

### OBJECT OF THE INVENTION

It is an object of the present invention to provide a method of manufacturing a porous polymer component with which it is possible to obtain a controlled and reproducible inner structure of the component. This may include a manufacturing which results in an inner structure that varies through the thickness of the component in a controlled manner.

It is another object of the present invention to provide a method of manufacturing a porous polymer component which removes the need for cleanroom facilities necessary for many known methods.

It is another object of the present invention to provide a method of manufacturing a porous polymer component with which it is easy to scale the dimensions of both the outer geometry of the component and the inner pores or channels to a desired size for a given application.

It is an object of at least some embodiments of the present invention to provide a method of manufacturing a porous polymer component having a structured inner structure through the thickness of the component.

It is another object at least some embodiments of the present invention to provide a method of manufacturing a porous polymer component which are more simple and efficient than known methods and which may therefore be advantageous especially for components to be produced fast or in low numbers.

It is a further object of the present invention to provide an alternative to the prior art.

### SUMMARY OF THE INVENTION

The above described object and several other objects are intended to be obtained in a first aspect of the invention by providing a method of manufacturing a porous polymer component with structured and/or random pores and/or channels, the method comprising the steps of:
- arranging a dissolvable, sacrificial material in a geometrical arrangement corresponding to an inner structure to be obtained in the polymer component,
- arranging a component material so that it surrounds at least a majority of the sacrificial material, and
- dissolving and removing the sacrificial material from the component material,
wherein the step of arranging the sacrificial material and thereby the resulting inner structure of the component is done in a controlled and reproducible manner.

By "porous component" is preferably meant that the component has a lot of very small holes in it so that a fluid, such as air or water, can pass through it.

By "pores" is preferably meant small openings or holes in a solid substance.

By "channels" is preferably meant a passageway along which something, in the present application typically a fluid, may pass or interconnected pores making such passage possible.

By "structured" is preferably meant that the pores or channels are arranged in an orderly and definable manner with respect to physical characteristics, such as dimensions or orientations.

By "random" is preferably meant that some characterising parameters relating to the geometrical arrangement in the inner structure, typically including the spatial orientation of the cavities, is not controllable. The inner structure of the component is still controlled in the sense that at least one parameter has been controlled to an extent so that it can be reproduced in a controlled manner. Such a parameter may e.g. be an average diameter of the pores or channels.

By "controlled" is preferably meant that at least some of the parameters which can be used to describe the porous structure are controllable and thereby also reproducible. Such parameters can e.g. be the volume fraction of solid material, an average size of the pores, or an average orientation of channels.

In some embodiments of the invention, the above-mentioned steps take place one at a time and in the order in which they are mentioned. In other embodiments some of the steps happen concurrently as will be explained below.

The method may further comprise the step of curing the component material before the sacrificial material is removed. This will be the case for materials which need curing before being in their final form and ready for handling. This will be explained in further details below. The sacrificial material may also be cured.

In some embodiments of the invention, the inner structure of the component is controlled to be random substantially throughout the component. Careful design of a random structure can be used to increase the surface-to-volume-ratio which will be advantageous for some applications of the invention. It is e.g. considered to be advantageous for applications within environmental engineering, such as for spongy material used to soak-up oil spill, or other applications exploiting the effect of surface tension.

The inner structure of the component may alternatively or in combination therewith be controlled to have a predefined geometrical configuration with orderly arranged pores and/or channels.

An example of an advantage of having both random and structured regions in a component is that in order to maximise mass transport in e.g. tissue engineering scaffolds, bigger channels enable rapid transport of fluid or nutrients while the smaller pores provide a larger functional surface area e.g. for cell growth, catalytic reactions, and adsorption or absorption or release of components over time. To summarise, having pores in combination with larger channels would enable access to a larger volume of the component.

An advantage of the possibility of controlling the sacrificial material so that structured channels are obtained is that it is possible to make the channels continue towards the centre of the component. This can e.g. be used in tissue engineering to make sure that also cells located at the centre are supplied with oxygen. The channels do not necessarily have the same size throughout the thickness of the component, but may resemble the decrease in size down to capillary in natural organs.

The inner structure of the component may be so that it comprises pores and/or channels of predefined dimensions through the thickness of the component.

The pores and/or channels may be of varying dimensions through the thickness of the component. The dimensions may e.g. vary from an outer surface of the component towards an inner region. Such a structure may e.g. find use within tissue engineering for the manufacturing of artificial organs. A graded structure in the form of a decreasing diameter of the channels towards the middle of the component can resemble the veins, arteries and/or blood vessels in a natural organ and be used to ensure that oxygen can be transported to and from a central region. It will also be possible to have one substantially constant value of a characteristic parameter, such as the average diameter, at some regions, such as near the surface, and another substantially constant value of the characteristic parameter at other regions, such as at the middle of the component.

In some embodiments of the invention the sacrificial material may be arranged by use of 3D-printing. 3D-printing is at present a quickly developing technology which can be used to precisely manufacture highly specialized structures. The obtainable dimensions and precision will depend on the 3D-printing technology used. At present it is in the order of micrometres and upwards. Hereby complex geometries of what are to become the network of porous and channels can be made in an easily controllable manner. In principle any printable and dissolvable material can be used; some examples will be mentioned below. The choice will depend both on the application of the final component and on the manufacturing method used.

By use of 3D-printing it is possible to make components in a wide range of dimensions. This technology is highly advantageous over the presently used methods as it is rapid and flexible with respect to both sizes and materials used. A further advantage is that the process can easily be automated.

In some embodiments of the invention, the sacrificial material and the component material are arranged concurrently by a use of 3D-printing. Hereby complex geometries can be built including some where part of the material for the final component needs to be supported by the sacrificial material during manufacturing until it is in a stable and self-supporting form.

In the embodiments of the invention including the use of 3D-printing, fused deposition technology may be used.

In the method as described above, two or more sacrificial materials may be arranged at different regions of the component being manufactured, the two or more sacrificial materials being dissolvable by use of different solvents, the method further comprising the step of sequentially dissolving and removing the two or more sacrificial materials from the component material. Hereby it will be possible to form more complex inner structures or outer geometries of the component.

The method may further comprise a subsequent step of pyrolyzing the component provided that a material which can be pyrolyzed is used. Examples of materials with which this step is possible are polymers such as polydimethyl siloxane (PDMS), polystyrene, block copolymers and different photo-curable carbon containing polymers. The pyrolyzed scaffold is a hard material which is suitable as a scaffold material for tissue engineering tissues, such as bone.

The component may be pyrolyzed so that an electrically conductive component is obtained. Such a component may e.g. be used for scaffolds for tissue engineering so that the conductivity allows the monitoring of cell growth and the potential for stimulating cells by electrical methods. Parameters that are relevant to control during the pyrolysis process are the temperature ramping speed, maximum temperature, time and combination of gases.

For some applications of the invention, an alternative to using 3D-printing for the manufacturing will be a method in which the sacrificial material is arranged manually with respect to a template adapted to assist in holding the filaments in a desired arrangement during manufacturing of the component. Such a manual arrangement of the sacrificial material may e.g. be winding of one or more filaments around the template. The filaments may e.g. be made by 3D-printing or by extrusion. This method can e.g. be used for the manufacturing of microfluidic lab-on-a-chip as will be shown in relation to the figures. With this method it is possible to make any type of geometrical features, such as channels with a circular cross section, which are difficult to make with other methods, including 3D-printing. The cross sectional dimensions of such filaments are typically in the micrometre range and upwards.

The sacrificial material may be made from any polymer which is dissolvable in water or another suitable solvent which does not damage the component material. It may e.g. be made from poly(vinyl alcohol), PVA, which is soluble in water or from wax. Other examples are acrylonitrile butadiene styrene (ABS) which is soluble in acetone and polylactic acid or polylactide (PLA). In the embodiments involving 3D-printing, the material chosen should be printable with the actual printer being used.

The component material may be selected from the following group: collagen, gelatine, polyhema, SU8, polystyrene, or silicone elastomers, such as polydimethylsiloxane (PDMS). SU8 is a commonly used epoxy-based negative photoresist which is found suitable for the present invention. It is in principle possible to use any artificial or natural polymers provided that they are applicable for the precise manufacturing method used. Some of the embodiments of the invention relate to polymers which can be cured, e.g. under the influence of light, to form a cross-linked material. Other embodiments relate to thermoplastic polymers which are heated to become liquid before being moulded around the sacrificial material and then cooled to solidify, preferably before the sacrificial material is removed. Gelatine is an example of a polymer which can be used both with and without the crosslink-forming curing step. The choice of component is highly dependent on the application of the component being manufactured. E.g. it is important for some applications that the component material is biocompatible, whereas for other applications it is necessary to use a material which can be pyrolyzed.

In a second aspect the invention relates to a polymer component obtained by a method as described above. Such a component may find use in a number of applications as will be described in further details below.

In a third aspect the invention relates to the use of a component manufactured as described above as a three-dimensional scaffold for tissue engineering. An example of such a use relates to the ability to fabricate a 3D tissue engineering scaffold including domains (the porous regions) for culturing effector cells such as hepatocytes and providing artificial veins, arteries and/or blood vessels (structured part) feeding these domains.

In a fourth aspect the invention relates to the use of a component manufactured as described above as a medical device. Such medical devics could e.g. be artificial organs and tissues as well as lab-on-a-chip systems. This fourth aspect of the invention also relates to a medical device comprising a polymer component as described above or to a component manufactured as described above for use as a medical device.

A fifth aspect of the invention is the application of these structures for cultivating a high cell density. This can be used for expanding different cell types or production of e.g. recombinant proteins.

The first, second, third, fourth and fifth aspect of the present invention may each be combined with any of the other aspects. These and other aspects of the invention will be apparent from and elucidated with reference to the embodiments described hereinafter.

### BRIEF DESCRIPTION OF THE FIGURES

The method according to the invention will now be described in more detail with regard to the accompanying figures. The figures show one way of implementing the present invention and is not to be construed as being limiting to other possible embodiments falling within the scope of the attached claim set.
Figure 1 shows schematically the overall steps in an embodiment of the invention used to manufacture a component with a substantially uniform inner structure throughout the component.
Figure 2 shows schematically examples of structures with pores and channels, respectively.
Figure 3 shows schematically the differences between structured and random inner structures in a component.
Figure 4 shows schematically the steps in a manufacturing method including a step of pyrolyzing the polymer component.
Figure 5 shows SEM images of a pyrolyzed component.
Figure 6 shows schematically an embodiment of the invention in which the sacrificial material is arranged manually.

### DETAILED DESCRIPTION OF AN EMBODIMENT

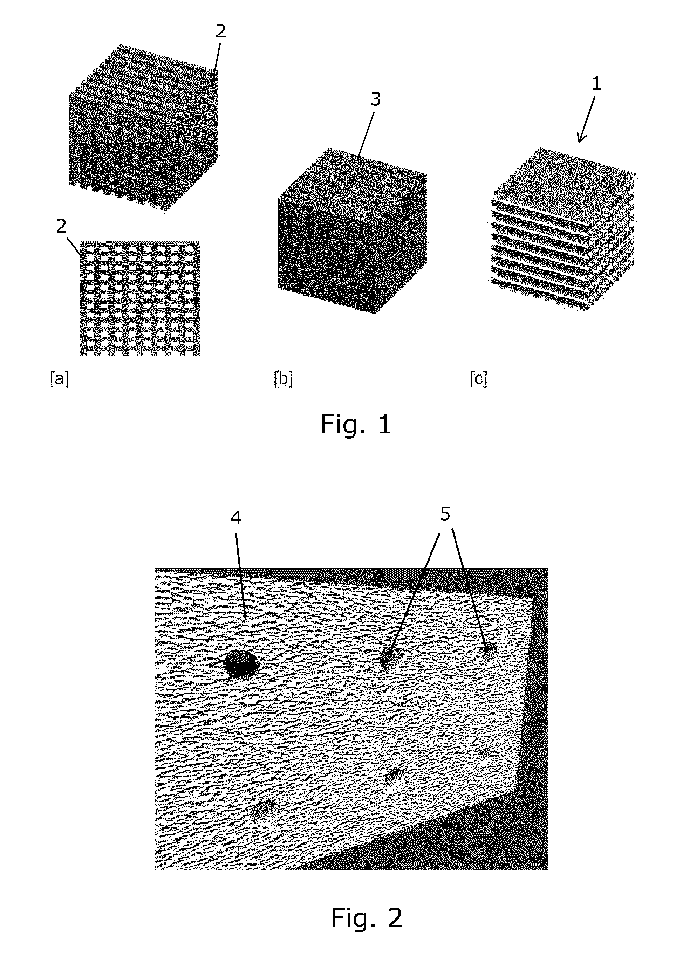

Figure 1 shows schematically the overall steps in an embodiment of the invention used to manufacture a porous polymer component 1 with a substantially uniform inner structure throughout the component 1. In this embodiment, the inner structure of the component 1 is controlled to have a predefined geometrical configuration with orderly arranged channels. Figure 1.a shows the dissolvable, sacrificial material 2 arranged in a geometrical arrangement corresponding to an inner structure to be obtained in the polymer component 1. The upper part of figure 1.a is a three-dimensional view, and the lower part if figure 1.a is a cross sectional view. Figure 1.b shows the component material 3 which has been arranged so that it surrounds a majority of the sacrificial material 2. Figure 1.c shows the final polymer component 1 after the sacrificial material 2 has been dissolved and removed from the component material 3. An important part of the process is that the arrangement of the sacrificial material 2 and thereby the resulting inner structure of the cavities, including the pores and/or channels, in the component 1 is done in a controlled and reproducible manner. How this can be done will be explained in further details below. Depending on the actual polymer material used, the method may further comprise the step of curing the component material 3 before the sacrificial material 2 is removed. This will e.g. be necessary in the case of polydimethyl siloxane (PDMS) as a component material 3. In the case of 3D-printing two or more different polymers such as PLA and ABS, the curing occurs in the same moment as the polymers are printed.

Figure 2 shows schematically examples of structures with pores 4 and channels 5, respectively. The geometries and relative sizes of the pores 4 and channels 5 in figure 2 are examples and given for illustrative purposes only. In a real component 1, e.g. the walls of the channels 5 may be rough, and the overall path of the channels 5 need not be along a straight line. The structure of the component 1 may be so that it comprises pores 4 and/or channels 5 of predefined dimensions through the thickness of the component 1. The channels 5 may also be of varying dimensions through the thickness of a component 1, such as from an outer surface of the component 1 towards an inner region of the component 1.

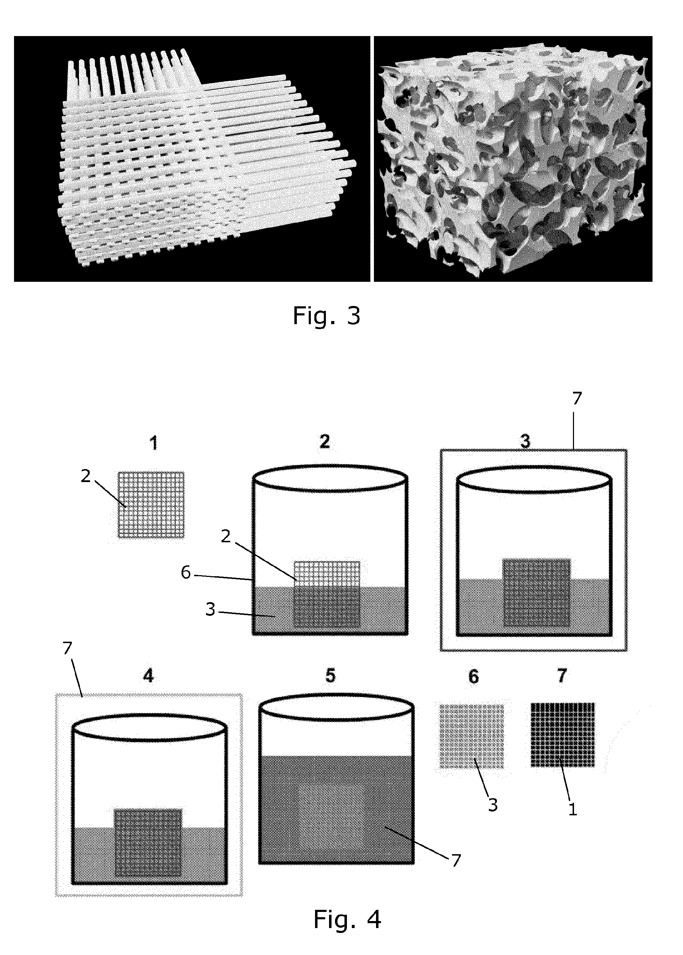

Figure 3 shows schematically the differences between structured (shown to the left) and random (shown to the right) inner structures in a component 1. It will also be possible to have regions with different structures in the component, such as structured regions with different cross sectional dimensions of the channels 5 or with different volume fractions of solid material. It will also be possible to have both random and structured regions in one component.

At least for some geometries and/or combinations of materials it will be advantageous to 3D-print both the sacrificial material 2 and the component material 3 concurrently. Hereby the pores 4 or channels 5 in the geometrical arrangement of the sacrificial material 2 are filled with the component material 3 while the component 1 is being formed. A typically used 3D-printing method is fused deposition technology.

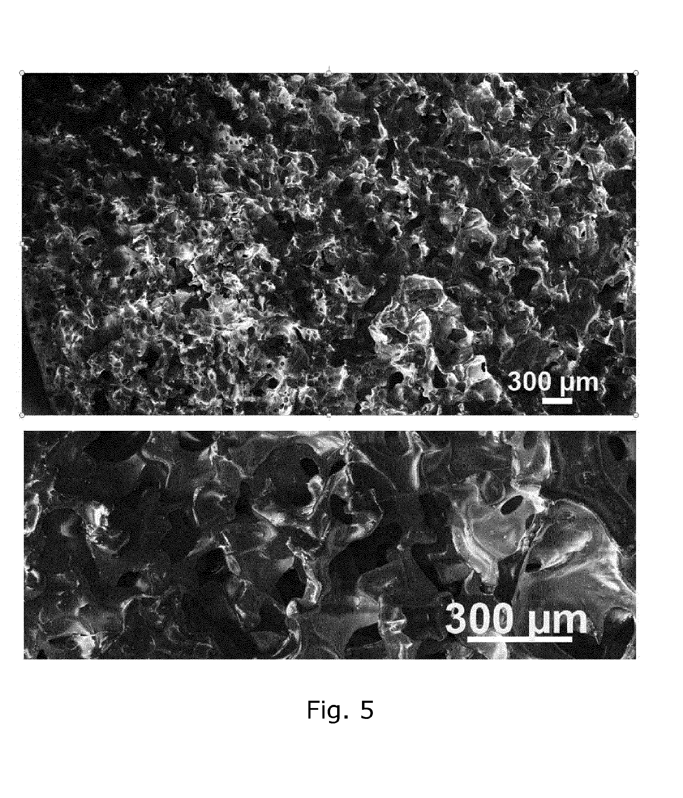

A method as described above may further comprise a subsequent step of pyrolyzing the component 1. This method step can e.g. be used to obtain an electrically conductive component which may e.g. find use in tissue engineering and sensing applications. Figure 4 shows schematically the steps in a process as described above including the step of pyrolyzing the component. 1: A sacrificial material 2 is provided e.g. by 3D-printing. 2: The sacrificial material 2 is placed in a container 6 with component material 3. 3: Vacuum is applied, e.g. in a vacuum chamber 7, to ensure that the component material 3 infiltrates the sacrificial material 2. 4: The component material 3 is then cured in an oven 8 at raised temperature for a predetermined time period. 5: The sacrificial material 2 is dissolved by placing the cured intermediate component in a solvent 9 for a predetermined time period or until the sacrificial material 2 has been dissolved and can be removed e.g. by washing (not shown). 6: The component 1 now being constituted by component material 3 can further be pyrolized to obtain the pyrolized final component 1 shown as number 7. Figure 5 shows SEM images of an example of such a pyrolized component at two different magnifications.

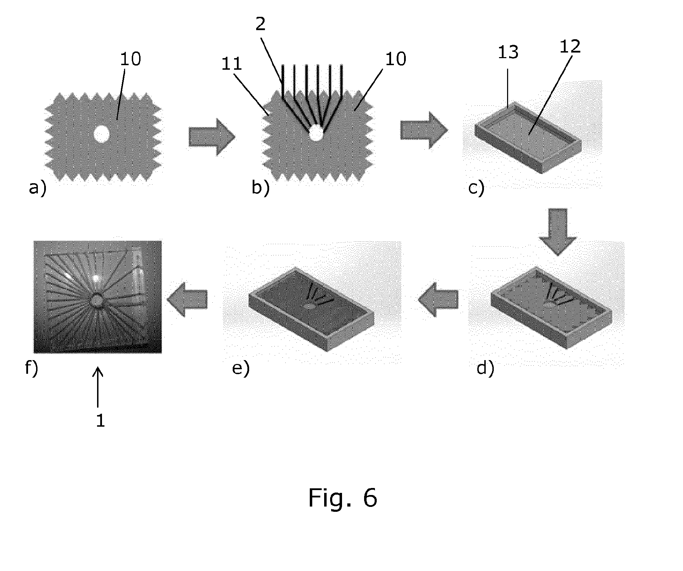

An alternative to 3D-printing which will be possible for some application is to arrange the sacrificial material 2 manually. Such a method can e.g. be used to manufacture a microfluidic device without the need for cleanroom fabrication technologies. Another advantage over some known methods is the possibility to form completely cylindrical channels 5, e.g. from arrangement of filaments having a circular cross section. An example of a method wherein the sacrificial material 2 is arranged manually is shown schematically in figure 6. Figure 6.a shows a frame 10, also referred to as a template, which can e.g. be made by laser ablation of a 1 mm thick PC sheet. As shown in figure 6.b, filaments of sacrificial material 2 are arranged around the template 10, and the grooves 11 along the edges of the template 10 assist in holding the filaments 2 in a desired arrangement during manufacturing of the component 1. Such filaments 2 can e.g. be 400 µm thick PVA filaments pre-made e.g. by extrusion or by 3D-printing. A cast and partially cured layer 12 of e.g. PDMA is arranged in a mould 13 as shown in figure 6.c, and the frame 10 with the filaments 2 are placed on top thereof as shown in figure 6.d. An upper layer of PDMS (not visible in the figure) in then is cast on top thereof as shown in figure 6.e, and the whole arrangement is cured, typically by application of heat or light depending of the type of material used. In this embodiment the component material 3 is constituted by the two layers 12 of PDMS and the template 10 which becomes cast into the component 1. After curing of the component material 3, it is removed from the mould 13, and the sacrificial filament material 2 is dissolved e.g. by placing the component 1 in hot water (not shown). The component may also be cut to a final shape as shown in figure 6.f.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. Method of manufacturing a porous polymer component (1) with structured and/or random pores (4) and/or channels (5), the method comprising the steps of:
- arranging a dissolvable, sacrificial material (2) in a geometrical arrangement corresponding to an inner structure to be obtained in the polymer component (1),
- arranging a component material (3) so that it surrounds at least a majority of the sacrificial material (2), and
- dissolving and removing the sacrificial material (2) from the component material (3),
wherein the step of arranging the sacrificial material (2) and thereby the resulting inner structure of the component (1) is done in a controlled and reproducible manner.

2. Method according to claim 1, further comprising the step of curing the component material (3) before the sacrificial material (2) is removed.

3. Method according to claim 1 or 2, wherein the inner structure of the component (1) is controlled to be random.

4. Method according to claim 1 or 2, wherein the inner structure of the component (1) is controlled to have a predefined geometrical configuration with orderly arranged pores (4) and/or channels (5).

5. Method according to claim 4, wherein the inner structure of the component (1) is so that it comprises pores (4) and/or channels (5) of predefined dimensions through the thickness of the component (1).

6. Method according to claim 5, wherein the pores (4) and/or channels (5) are of varying dimensions through the thickness of the component (1).

7. Method according to any of the preceding claims, wherein the sacrificial material (2) is arranged by use of 3D-printing.

8. Method according to claim 7, wherein both the sacrificial material (2) and the component material (3) are arranged concurrently by a use of 3D-printing.

9. Method according to claim 7 or 8, wherein fused deposition technology is used.

10. Method according to any of the preceding claims, wherein two or more sacrificial materials (2) arranged at different regions of the component (1) being manufactured, the two or more sacrificial materials (2) being dissolvable by use of different solvents (9), the method further comprising the step of sequentially dissolving and removing the two or more sacrificial materials (2) from the component material (3).

11. Method according to any of the preceding claims, further comprising a subsequent step of pyrolyzing the component (1).

12. Method according to claim 11, wherein the component (1) is pyrolyzed so that an electrically conductive component is obtained.

13. Method according to claim 1, wherein the sacrificial material (2) is arranged manually with respect to a template (10) adapted to assist in holding the sacrificial material (2) in the form of filaments in a desired arrangement during manufacturing of the component (1).

14. Use of a component (1) manufactured according to any of the preceding claims as a three-dimensional scaffold for tissue engineering.

15. Use of a component (1) manufactured according to any of claims 1 to 13 as a medical device.
